# FASCICULE DE BREVET EUROPEEN

(11) **EP 1 054 697 B1**
(45) Date de publication et mention de la délivrance du brevet: **11.12.2002**
(21) Numéro de dépôt: 99900991.3
(22) Date de dépôt: 22.01.1999
(51) Int. Cl.: A61L 9/12

(54) **DIFFUSEUR DE SUBSTANCE VOLATILE SANS REMANENCE**
VERDUNSTER FÜR FLÜCHTIGE SUBSTANZEN OHNE REMANENZ
VOLATILE SUBSTANCE DIFFUSER WITHOUT PERSISTENCE

(30) Priorité: 10.02.1998 FR 9801526
(43) Date de publication de la demande: 29.11.2000
(73) Titulaire: ATELIERS DE CONCEPTIONS ET D'INNOVATIONS INDUSTRIELLES, F-92400 Courbevoie (FR); Pozzo, Michel, 92200 Neuilly-sur-Seine (FR)
(72) Inventeur: POZZO, Michel, F-92200 Neuilly-sur-Seine (FR)
(74) Mandataire: Texier, Christian
(86) Numéro de dépôt international: FR9900135
(87) Numéro de publication internationale: WO99040950

(56) Documents cités:
- EP-A- 0 104 758
- DE-U- 29 510 619
- FR-A- 1 500 142
- US-A- 4 146 566
- US-A- 5 014 913

## Description

L'invention concerne les diffuseurs de substance volatile.

On connaît d'après le brevet EP-0 104 758 un diffuseur de substance volatile comprenant un corps et une cartouche d'un produit saturé au moyen d'une substance volatile. Le corps peut recevoir la cartouche de façon amovible. Un radiateur permet de chauffer de l'air sous la cartouche pour le forcer à traverser la cartouche et à s'échapper par une ouverture supérieure du corps pour diffuser la substance. Pour interrompre la diffusion, il faut ôter la cartouche du corps, puis obturer hermétiquement la cartouche au moyen d'un organe de protection. Ces opérations sont relativement longues à réaliser. En outre, la substance continue à diffuser lorsque la cartouche est ôtée du corps et tant qu'elle n'est pas revêtue de l'organe de protection. La diffusion cesse donc très progressivement pendant une période de temps assez longue et notamment après que l'utilisateur a souhaité l'interrompre. Il se produit ainsi un effet de rémanence olfactive et il faut de préférence attendre la disparition totale de cet effet avant de diffuser une autre substance.

On connaît par ailleurs du document FR-1 500 142 un diffuseur d'odeur comprenant deux tubes cylindriques concentriques présentant des ouvertures latérales et mobiles à rotation pour mettre à volonté leur ouvertures en coïncidence pour diffuser l'odeur d'une substance logée dans le tube interne. Avec ce dispositif, supprimer l'effet de rémanence olfactive suppose la disposition entre les deux tubes d'un joint étanche à l'air. Or, ce joint a une forme relativement compliquée à réaliser.

Un but de l'invention est de fournir un diffuseur permettant d'interrompre brutalement la diffusion et facile à fabriquer.

En vue de la réalisation de ce but, on prévoit selon l'invention un diffuseur de substance volatile comportant un corps présentant au moins une ouverture, et un réservoir d'un produit comprenant une substance volatile, le réservoir étant monté mobile dans le corps entre une position de diffusion permettant qu'un gaz circule à travers le produit, puis dans l'ouverture et une position fermée dans laquelle le produit est isolé de l'ouverture, le réservoir et le corps présentant des faces respectives s'étendant en regard l'une de l'autre, dans lequel l'une des faces est sphérique, et qui comporte au moins un élément assurant une étanchéité entre le corps et le réservoir dans la position fermée.

Ainsi, en présence d'une face sphérique, le joint d'étanchéité peut avoir une forme simple à réaliser, par exemple une forme circulaire. L'étanchéité étant totale, l'odeur n'est pas du tout diffusée en position fermée. La disposition du réservoir en position fermée permet d'interrompre brusquement la diffusion de la substance volatile, sans effet de rémanence. On peut donc alterner rapidement les diffusions de substances différentes. De plus, l'interruption de la diffusion est simple et rapide à exécuter.

Avantageusement, la face sphérique est celle du réservoir.

Avantageusement, le réservoir est mobile à rotation par rapport au corps.

Avantageusement, le réservoir est mobile pour tourner sur lui-même de sorte que le réservoir occupe un même emplacement dans le corps dans la position de diffusion et la position fermée.

Ainsi, le changement de position est compatible avec un faible encombrement dans le corps.

Avantageusement, le réservoir est mobile autour d'un axe de rotation perpendiculaire à une direction de circulation du gaz à travers le produit.

Avantageusement, les 'faces présentent chacune une symétrie de révolution autour de l'axe de rotation.

Avantageusement, les faces sont sphériques.

Avantageusement, le réservoir présente une face interne sphérique.

Avantageusement, l'ouverture étant une première ouverture, le corps présente une deuxième ouverture adaptée pour qu'en position de diffusion, le gaz circule dans la deuxième ouverture, puis à travers le produit, puis dans la première ouverture.

Avantageusement, le réservoir comporte une cartouche amovible comprenant le produit.

Avantageusement, le produit comporte des granulés comprenant la substance volatile à l'état adsorbé.

Les granulés peuvent adsorber une grande quantité de substance volatile. Les espaces entre les granulés permettent d'offrir une grande surface d'échange entre le gaz circulant et les granulés pour la diffusion de la substance.

Avantageusement, le réservoir comporte une paroi présentant des orifices ayant chacun un contour conformé de sorte qu'il ménage un passage de gaz lorsqu'un granulé sphérique est en appui sur l'orifice.

Ainsi, la paroi maintient les granulés quelle que soit la position de la paroi, par exemple si elle est verticale, et les orifices ne sont pas obturés par les granulés.

Avantageusement, les orifices ont un contour circulaire gauche.

Avantageusement, la paroi est ondulée.

Ainsi, on donne une forme gauche aux orifices de façon particulièrement simple.

On prévoit également selon l'invention un dispositif de diffusion de substance volatile comprenant au moins un diffuseur selon l'invention, et un support adapté à recevoir le diffuseur ou les diffuseurs simultanément.

Avantageusement, le support est adapté à retenir le ou chaque diffuseur par encliquetage.

Avantageusement, le support comporte des moyens pour commander la position du réservoir du ou de chaque diffuseur.

Avantageusement, le support comporte des moyens pour mettre un gaz en circulation à travers le ou chaque diffuseur en position de diffusion.

Il peut s'agir d'un ventilateur, ce qui évite d'échauffer les produits et de dégrader les substances à diffuser, ou mieux encore d'une turbine qui présente l'avantage supplémentaire d'être plus silencieuse.

Avantageusement, le support est par exemple un orgue et comprend au moins deux diffuseurs, les moyens de circulation de gaz étant communs aux diffuseurs.

Avantageusement, le dispositif de diffusion comporte des moyens de détrompage pour la réception du diffuseur dans au moins une position prédéterminée dans le support.

Avantageusement, le dispositif de diffusion comporte des moyens de détrompage pour la réception dans le support d'au moins un diffuseur prédéterminé.

D'autres caractéristiques et avantages de l'invention apparaîtront encore dans la description qui va suivre de deux modes préférés de réalisation donné à titre d'exemples non limitatifs. Aux dessins annexés :
- la figure 1 est une vue en coupe axiale et en perspective d'un corps de diffuseur selon un premier mode de réalisation de l'invention ;
- la figure 2 est une vue en perspective d'un boisseau du diffuseur de la figure 1 ;
- la figure 3 est une vue en coupe axiale montrant le corps et le boisseau des figures 1 et 2 assemblés en position fermée ;
- la figure 4 est une vue en plan de la paroi de la cartouche de la figure 3 ;
- la figure 5 est une vue en coupe selon le plan V-V de la paroi de la figure 4 ;
- la figure 6 est une vue en perspective d'un dispositif de diffusion supportant des diffuseurs selon la figure 3 ;
- la figure 7 est une vue en coupe transversale du dispositif de la figure 6 ;
- les figures 8 et 9 sont des vues respectives de dessus en coupe et de droite en coupe d'un support pour un dispositif de diffusion selon un deuxième mode de réalisation de l'invention ;
- les figures 10 et 11 sont des vues respectives de droite et de face du boîtier d'un diffuseur destiné à être reçu dans le support des figures 8 et 9 ;
- les figures 12, 13 et 14 sont des vues en coupe du boîtier du diffuseur selon les plans XII-XII, XIII-XIII et XIV-XIV des figures 10 et 11 ;
- les figures 15 et 16 sont des vues respectives en coupe axiale et de face d'un flasque d'obturation du boîtier des figures 10 à 14 ;
- les figures 17, 18 et 19 sont des vues de dessous, de face et en coupe axiale d'un boisseau à recevoir dans le boîtier des figures 10 à 14 ;
- la figure 20 est une vue en coupe axiale illustrant le boisseau de la figure 19 entre les deux flasques de la figure 15 ;
- les figures 21 à 24 sont des vues de face, en coupe axiale, de dessus et de dessous de l'arbre d'entraînement du diffuseur de la figure 10 ;
- les figures 25 et 26 sont des vues en élévation partiellement en coupe axiale, et de dessus, de l'arbre d'entraînement du support de la figure 8 ;
- la figure 27 est une vue en coupe axiale d'un joint du diffuseur de la figure 10 ;
- la figure 28 est une vue analogue à la figure 20 illustrant une variante de réalisation ; et
- la figure 29 est un schéma d'un circuit électronique de commande du support.

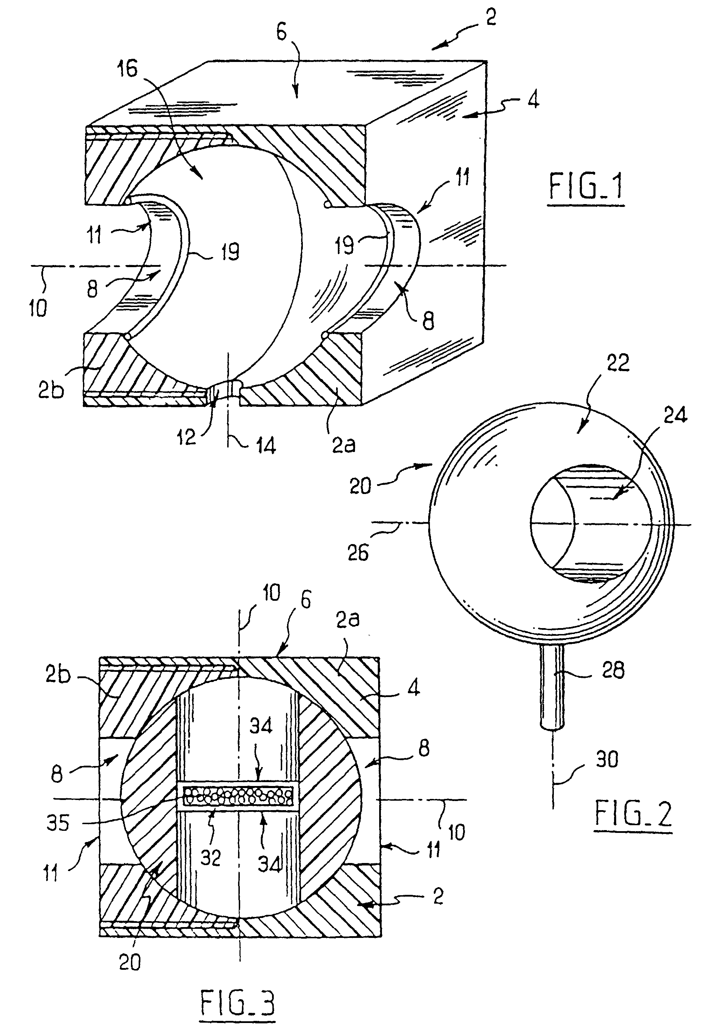

En référence aux figures 1 et 3, un diffuseur 2 selon le présent mode de réalisation de l'invention comporte un corps 4 ayant une face externe 6 de forme quelconque, par exemple cubique. Le corps 4 présente un conduit cylindrique 8 d'axe 10, débouchant à deux faces opposées avant et arrière du corps en définissant deux ouvertures avant et arrière 11 et passant par le centre du corps. Le corps 4 présente en outre un conduit cylindrique 12 d'axe 14 perpendiculaire à l'axe 10 et coupant celui-ci géométriquement au centre du corps. Le conduit 12 débouche sur une face inférieure externe du corps.

Le corps 4 a une face interne 16 présentant une symétrie de révolution autour de l'axe 14. En l'espèce, cette face 16 est sphérique et a son centre au centre du corps. Elle coupe géométriquement les conduits 8 et 12. Le corps 4 présente deux joints sous la forme de cordons circulaires 19 en élastomère disposés sur la face sphérique 16 en étant contigus aux deux parties du conduit 8 séparées l'une de l'autre par la face sphérique 16.

En référence aux figures 2 et 3, le diffuseur 2 comporte en outre un boisseau 20 présentant une face externe sphérique 22 sensiblement de même rayon que la face interne sphérique 16 du corps 4, de sorte que le boisseau 20 peut être disposé dans le corps 4, les deux faces sphériques 16, 22 venant en regard l'une de l'autre. Le boisseau 20 présente un conduit cylindrique 24 d'axe 26 traversant la sphère diamétralement de part en part. Le boisseau 20 comporte un arbre 28 d'axe 30 orienté radialement par rapport au centre de la sphère et perpendiculairement à l'axe 26. L'arbre 28 s'étend en saillie de la face externe sphérique 22.

En référence aux figures 3 à 5, le boisseau 20 comporte une cartouche 32 présentant une face externe cylindrique en contact surface contre surface avec la face du conduit 24, et deux parois d'extrémité avant et arrière 34. Les parois 34 ont une forme circulaire généralement plate. Toutefois, chaque paroi 34 présente en profil transversal des ondulations. De plus, elle présente des orifices 36 de forme circulaire en vue en plan ménagés dans des portions localement non planes de la paroi. De la sorte, les orifices 36 ont des contours gauches, c'est-à-dire non plans. La cartouche 32 renferme un produit 35, ici sous la forme de granulés de matière plastique comprenant une substance volatile telle qu'un parfum à l'état adsorbé. Il peut s'agir par exemple de granulés de polymère commercialisés par ELF ATOCHEM sous la marque PEBAX. Le boisseau 20 et la cartouche 32 constituent ici le réservoir.

Le boisseau 20 est disposé dans le corps 4, l'arbre 28 s'étendant dans le conduit 12 et à l'extérieur du corps 4. Le boisseau 20 est mobile à rotation par rapport au corps 4 autour des axes 14 et 30 confondus. Le boisseau 20 occupe le même emplacement dans le corps quelle que soit sa position en rotation autour de l'axe 14. Une action sur l'arbre 28 permet de placer le boisseau 20 dans l'une quelconque des positions suivantes. En position ouverte, ou position de diffusion, les conduits 8 et 24 sont coaxiaux et dans le prolongement l'un de l'autre. Lorsque de l'air entre dans l'une des ouvertures 11, il traverse successivement et dans cet ordre une portion du conduit 8, une portion du conduit 24, une paroi d'extrémité 34 de la cartouche 32, le produit 35, l'autre paroi 34 d'extrémité de la cartouche, l'autre portion du conduit 24, l'autre portion du conduit 8 et la deuxième ouverture 11. Au passage entre les granulés 35, l'air se charge en parfum désorbé qu'il diffuse à travers la deuxième ouverture 11. Les ondulations des parois 34 de la cartouche 32 permettent de remplir celle-ci totalement avec des granulés sans que ceux-ci n'obturent les orifices 36. L'air peut donc traverser la cartouche de part en part.

En position fermée, le boisseau 20 s'étend avec l'axe 26 du conduit 24 perpendiculairement à l'axe 10 du conduit 8 du corps 4. La paroi du boisseau 20 s'étend en regard des deux portions du conduit 8 qu'elle obture. La cartouche 32 est isolée du corps 4 et des ouvertures 11, cette isolation étant étanche grâce aux joints 19.

On peut également avoir une position d'ouverture (ou de fermeture) partielle lorsque les axes 10 et 26 des conduits 8 et 24 sont légèrement inclinés l'un par rapport à l'autre. Dans cette position, seule une faible communication d'air est permise de l'une à l'autre des ouvertures 11 à travers la cartouche 32.

Pour permettre d'introduire le boisseau 20 dans le corps 2, celui-ci comprend par exemple une partie principale 2a évidée sur la face présentant la première ouverture 11 et présentant un logement intérieur fileté d'axe 10. Le corps 2 comprend en outre par ailleurs une partie rapportée 2b définissant la plus grande partie de la face présentant la première ouverture 11 et présentant une face externe cylindrique filetée adaptée à être visée dans le logement de la partie principale 2a. Chacune des deux parties principale 2a, et rapportée 2b définit un hémisphère de la face interne sphérique 16. On loge le boisseau 20 dans la partie principale 2a avant de rapporter sur celle-ci la partie rapportée 2b.

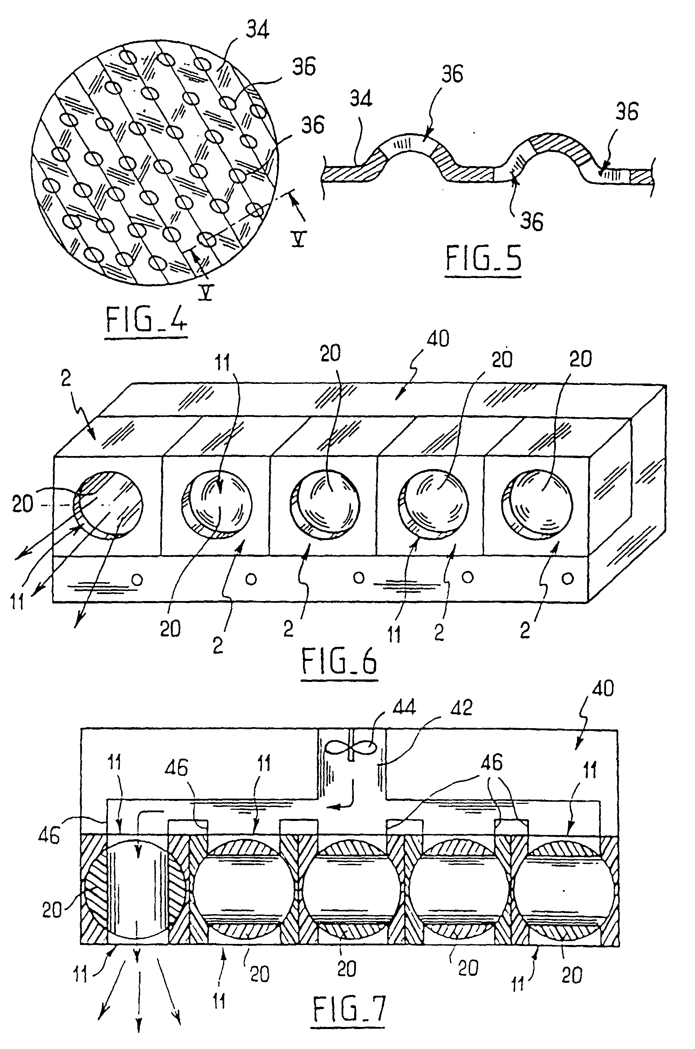

En référence aux figures 6 et 7, un dispositif de diffusion selon un mode préféré de réalisation de l'invention est un orgue à parfums et comporte un support 40 présentant une série d'emplacements adaptés à recevoir des diffuseurs 2 disposés côte à côte avec les ouvertures avant 11 orientées dans la même direction, la série d'emplacements constituant un module. Le support comprend des moyens de retenue par encliquetage des diffuseurs dans leurs emplacements. Chaque emplacement comprend un actionneur non représenté adapté à coopérer avec l'axe 30 du diffuseur 2 associé pour manoeuvrer le boisseau 20 par rapport au corps 4. Le support 40 comporte un canal primaire 42 comprenant un ventilateur 44 et raccordé à une série de conduits secondaires 46 débouchant aux emplacements respectifs en regard de l'ouverture arrière 11 des conduits 8 des diffuseurs. Le support 40 comporte des moyens pour commander la mise en marche du ventilateur 44 et pour choisir la position, fermée, partiellement ouverte, ou ouverte, de chacun des diffuseurs 2. Chacun des diffuseurs 2 comporte une cartouche 32 associée à une substance différente, par exemple un parfum. Les diffuseurs sont tous alimentés en air par le ventilateur 44, source d'air commune, puisant son air en partie arrière du support 40.

Lorsque tous les diffuseurs 2 sont fermés, pour obtenir la diffusion de l'un ou plusieurs des parfums, il suffit de mettre le ou les diffuseurs 2 associés en position d'ouverture partielle ou totale. Le ventilateur 44 alimente en air le ou les diffuseurs ouverts. La diffusion d'une substance volatile au moyen du diffuseur s'effectue sans élévation de température, ce qui évite de dégrader la substance à diffuser. Les diffuseurs fermés sont soumis par le ventilateur 44 à une légère surpression. Toutefois, chaque diffuseur 2 en position fermée interdit l'envoi de parfum même en cas de surpression.

Lorsqu'un diffuseur 2 initialement en position ouverte est placé en position fermée, la diffusion du parfum associé cesse. De plus, la cartouche 32 est isolée de l'extérieur du dispositif. Il ne se produit donc aucun phénomène de rémanence olfactive. De plus, le produit 35 comprenant le parfum est mis à l'abri et peut ainsi être conservé longtemps. Le support 40 comprendra avantageusement des moyens de détrompage pour le montage de chaque diffuseur 2 en bonne position dans son emplacement. On pourra prévoir des moyens connus en soi pour interdire la manoeuvre du diffuseur 2 pour la diffusion du parfum s'il n'est pas en place sur le support. La position d'ouverture partielle permet de doser l'intensité du parfum diffusé. Un moteur pas à pas permettra de choisir la position d'ouverture partielle de chaque diffuseur avec une grande précision.

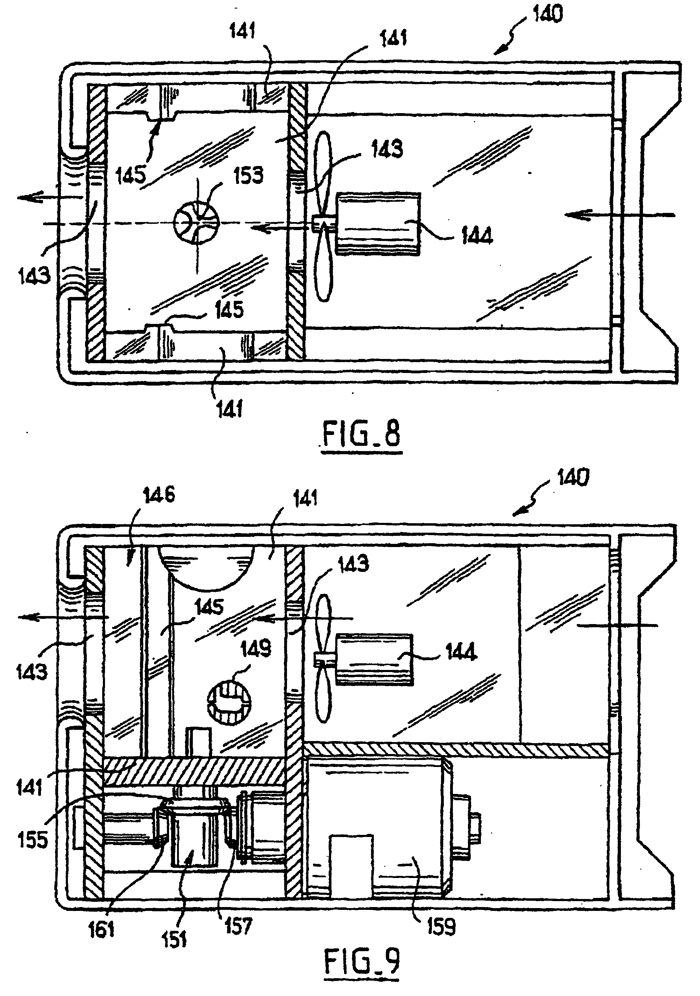

On va maintenant décrire un deuxième mode préféré de réalisation en référence aux figures 8 à 27 avec des références numériques augmentées de 100.

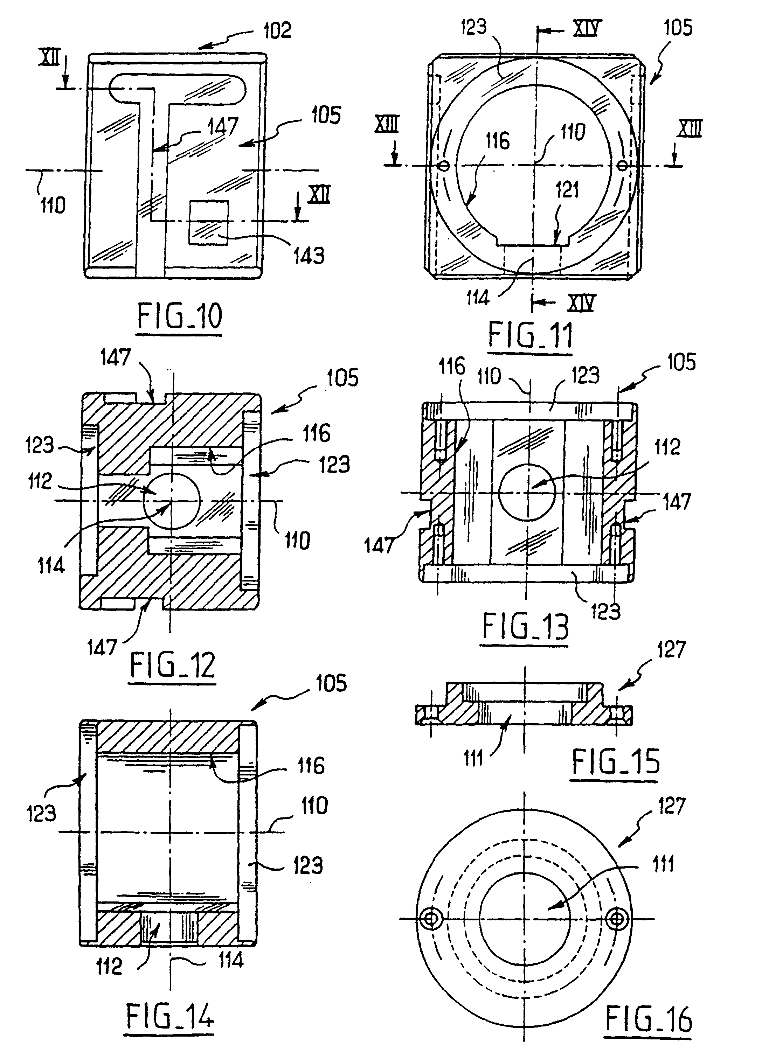

Le support 140 illustré aux figures 8 et 9 permet de recevoir un diffuseur 102 illustré aux figures 10 à 20. Le diffuseur 102 comporte un corps 104 comprenant une enveloppe creuse 105. Celle-ci a une forme externe de forme générale cubique et une forme interne de forme générale cylindrique. L'enveloppe 105 présente une face interne cylindrique 116 d'axe 110, et un orifice inférieur 112 d'axe 114, les axes 110 et 114 étant sécants et perpendiculaires entre eux. La face cylindrique 116 est interrompue par un méplat 121 s'étendant dans un plan perpendiculaire à l'axe 114 et traversé en son centre par l'orifice 112. L'enveloppe 105 présente à ses deux extrémités axiales deux logements de flasque 123. En référence aux figures 15 et 16, le corps 104 comporte deux flasques 127 constitués chacun par deux disques concentriques accolés pour former deux épaulements. Le disque de plus grand diamètre est destiné à occuper le logement 123 correspondant et le disque de plus petit diamètre pénètre dans la face cylindrique 116 qu'il obture. Le plus grand disque est fixé à l'enveloppe 105 par deux vis. L'enveloppe 105 et les flasques 127 présentent des emplacements de vis à cet effet. Les flasques forment les ouvertures 111.

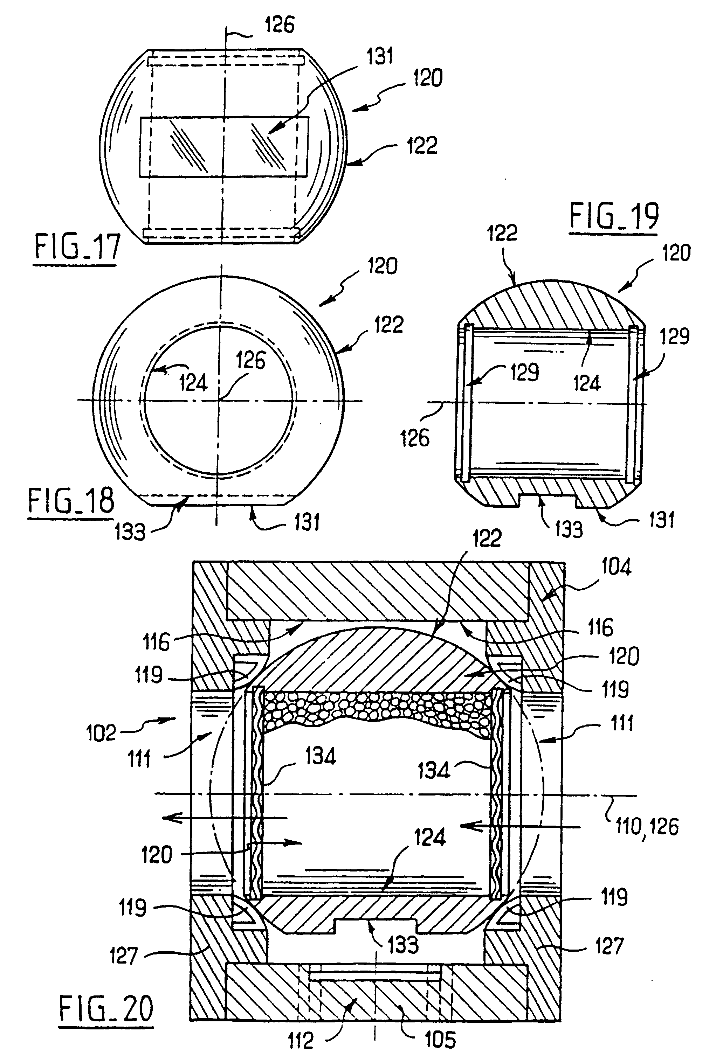

En référence aux figures 17 à 20, le diffuseur 102 comporte un boisseau 120 qui a ici encore une face externe sphérique convexe 122 et une face interne cylindrique 124 d'axe 126 coaxiale à celle-ci. La face interne cylindrique 124 présente au voisinage de ses extrémités axiales deux gorges annulaires 129. Les gorges 129 reçoivent respectivement deux grilles 134 identiques aux grilles 34 du premier mode de réalisation. Le boisseau 120 est rempli de granulés dans l'enceinte délimitée entre les deux grilles 134 par la face interne 124. C'est donc ici presque tout le volume interne du boisseau qui est occupé par les granulés. Le boisseau présente une face externe inférieure plane 131 parallèle à l'axe 126 et, dans celle-ci, une cavité à fond plat rectangulaire 133.

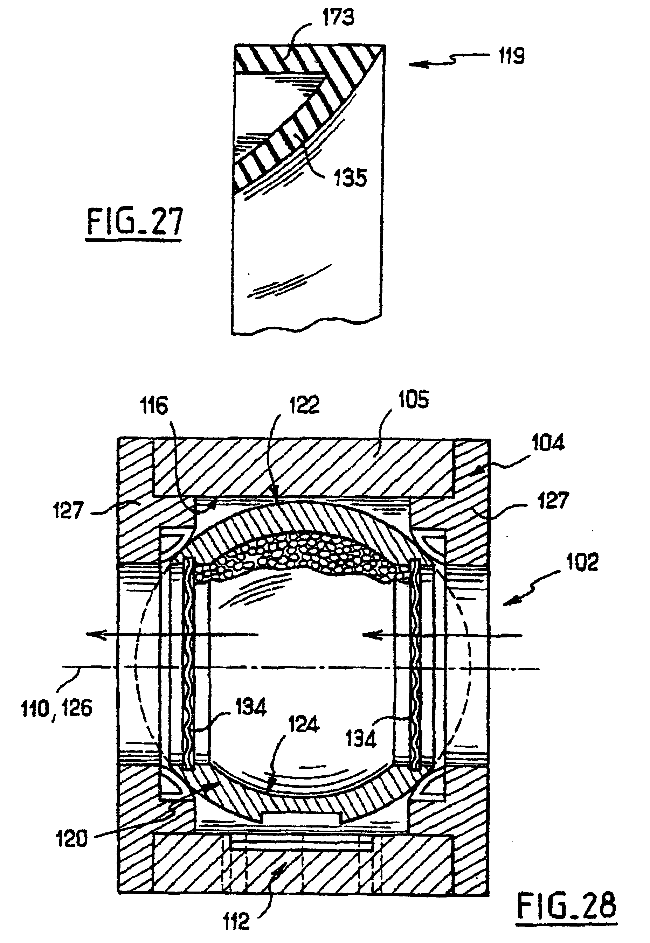

Comme illustré à la figure 20, le boisseau 120 est logé coaxialement dans le corps 104. La face sphérique externe 122 du boisseau s'étend en regard des flasques 127 et de la face cylindrique interne 116 de l'enveloppe. Chaque flasque présente un épaulement interne recevant un joint d'étanchéité 119 de forme annulaire circulaire représenté aux figures 20 et 27. Ce joint a un profil en « V ». Il comprend un pied 173 parallèle à l'axe 110 et une lèvre 135 reliée à un bord le plus interne du pied et localement parallèle à la face sphérique 122 contre laquelle elle vient en contact. En position ouverte illustrée sur la figure 20, les joints 119 s'étendent très près des grilles 134, autour de celles-ci. En position fermée, les grilles 134 s'étendent en regard de la face interne 116, entre les deux joints qui isolent ces grilles de l'extérieur.

En référence aux figures 8 et 9, le support 140 comporte des parois 141 délimitant un logement de réception du diffuseur 102, sensiblement de même forme que celui-ci, présentant deux ouvertures 143 destinées à être en regard des ouvertures 111 du diffuseur. Le logement présente une ouverture supérieure 146 pour l'introduction du diffuseur par coulissement vers le bas. Le support comporte un ventilateur 144 apte à envoyer de l'air dans l'ouverture amont 143. De préférence, pour réduire le bruit, ce ventilateur sera remplacé par une turbine.

Les parois latérales verticales 141 formant le logement présentent dans leur portion la plus proche de l'ouverture 143 aval une nervure verticale 145. Les parois latérales verticales du diffuseun 102 présentent de même une rainure 147 apte à recevoir la nervure 145 correspondante. Nervures et rainures constituent des moyens de détrompage pour l'introduction du diffuseur 102 dans le logement dans une position correcte prédéterminée.

Par ailleurs, le diffuseur 102 pourra comprendre des moyens d'identification électronique tels qu'une puce associée à des moyens de lecture 143 accessibles sur une face latérale du diffuseur et illustrés à la figure 10. Le support 140 comprendra alors un microprocesseur et des moyens de dialogue 149 avec les moyens de lecture 143 du diffuseur reçu dans le logement. Ces divers éléments électroniques constitueront des moyens d'identification électronique du diffuseur 102 reçu par le support 140 à des fins de détrompage lorsque, en fonction des circonstances d'utilisation du support 140, on souhaite que seulement certains diffuseurs 102 prédéterminés soient reçus dans le support. Ce sera le cas par exemple lorsque le support est utilisé aux fins de promotion d'un ou plusieurs parfums et ne doit en conséquence recevoir que le ou les diffuseurs correspondant à ce ou ces parfums.

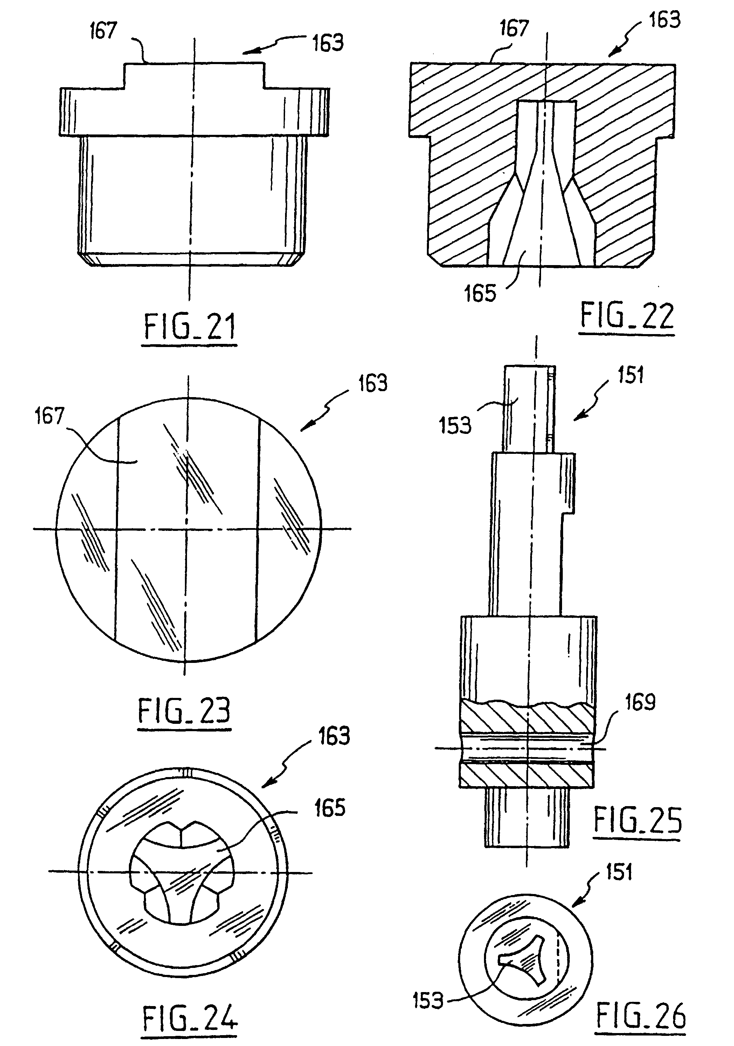

Dans le support 140, la paroi inférieure 141 formant le logement est traversée par un arbre d'entraînement 151 d'axe vertical dont une extrémité supérieure 153 à profil triangulaire à faces incurvées fait saillie dans le logement. Cet arbre est illustré aux figures 25 et 26. Sous la paroi 141, l'arbre 151 porte un pignon 155 engrenant avec un pignon 157 d'un arbre d'un moteur électrique 159 et un pignon d'équilibrage 161. Le diffuseur 102 comporte un arbre d'entraînement 163, illustré aux figures 21 à 24. Cet arbre 163 présente sur sa face inférieure une cavité 165 apte à recevoir l'extrémité supérieure 153 de l'arbre 151 du support 140 et à être entraîné en rotation par celle-ci. A son extrémité supérieure 167, l'arbre 163 du diffuseur comporte un relief plat s'étendant en saillie et en prise avec la cavité 133 du boisseau 120 pour entraîner celui-ci en rotation. Dans ces conditions, la rotation de l'arbre du moteur 159 est transmise à l'arbre 151 du support 140 puis à celui 163 du diffuseur et au boisseau 120 pour la commande de sa position. L'empreinte creuse 165 de l'arbre secondaire 163 limite la possibilité pour l'utilisateur de manoeuvrer le boisseau 120 lorsque le diffuseur 102 n'est pas reçu dans le support. L'arbre 151 du diffuseur pourra présenter un conduit cylindrique 169 perpendiculaire à son axe pour la réception d'une tige destinée à venir en butée contre des éléments du support pour définir les extrémités de la course en rotation de l'arbre 151.

Dans la variante de la figure 28, le boisseau 120 a une face interne sphérique concave 124 en vue de loger une plus grande quantité de granulés.

On pourra prévoir que l'un des deux flasques 127 est moulé d'une seule pièce avec l'enveloppe 105. On pourra également prévoir que l'un au moins des deux flasques est soudé, par exemple par ultrasons, à l'enveloppe pour interdire à l'utilisateur de remplacer les granulés, le diffuseur 102 étant alors prévu comme consommable.

Un potentiomètre de positionnement peut être adjoint au support 140 pour commander une ouverture partielle du diffuseur.

Le support 140, notamment lorsqu'il est à la disposition du public, pourra être prévu pour être commandé par un unique bouton poussoir. On pourra prévoir qu'une action sur ce poussoir déclenche la mise en marche du ventilateur 144 et simultanément l'ouverture du diffuseur. Une temporisation ferme le diffuseur et arrête le ventilateur à l'issue d'une période de temps prédéterminée réglable. Cette version est bien adaptée au test d'un parfum sur un lieu de vente.

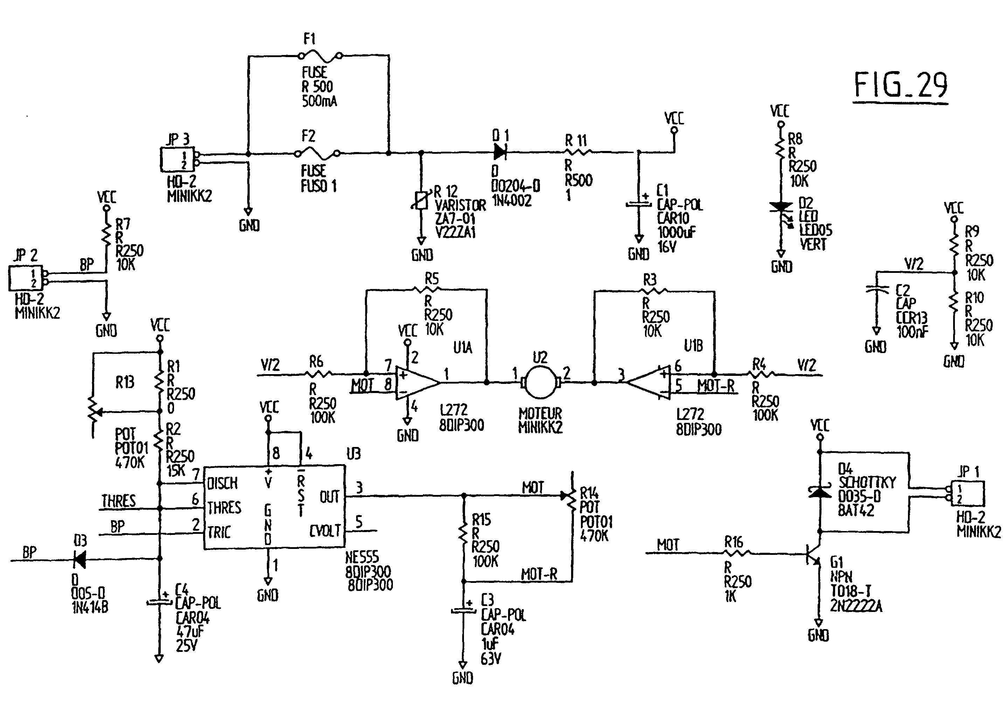

Un circuit de commande électronique pour le support 140 permettant un tel fonctionnement est illustré à la figure 29. Lorsque l'utilisateur appuie sur le bouton poussoir, le ventilateur se met en route, et la vanne s'ouvre. Lorsque le délai prévu (environ dix secondes) est écoulé, le ventilateur s'arrête et la vanne se referme. Si l'utilisateur appuie de nouveau sur le poussoir, le cycle recommence. De même, si l'utilisateur maintient le poussoir enfoncé, la durée du cycle est allongée.

La fonction timer est assurée par un NE555 (U3) qui est monté en monostable redéclenchable. La durée de temporisation T est réglée suivant la formule suivante : T = 0,693* (R13 + R12)* C4 avec C4 = 47 uF ; R2 = 16K ; R13 variable 0 à 470K.

La durée peut donc varier de 0,5 secondes à 15,8 secondes. Dans une version de série, le potentiomètre pourra être remplacé par une résistance R1 dont la valeur sera ajustée pour le temps désiré. La durée est allongée si l'on tourne le potentiomètre dans le sens horaire.

Le ventilateur 144 - est commandé via un amplificateur à transistor par la sortie du NE555. La diode D4 sert de roue libre pour limiter la surtension liée aux phénomènes inductifs.

Le moteur 159 est commandé par un amplificateur de puissance double L222 (U1). La durée d'alimentation du moteur est fixée par le réseau RC (R14, C4). Ce temps peut varier de 0 à 5 secondes.

Les composants D1 R11, C1, R12, F1 et F2 sont utilisés pour la protection du circuit.

D1 protège contre les inversions de polarité.

R11 et C1 forment un réseau RC de régulation des pointes de courant pour améliorer la compatibilité CEM.

R12 écrête les surtensions.

F1 ou F2 sont des fusibles pour la protection contre les courts-circuits. F1 est un fusible à filament qui met la carte hors fonction définitivement en cas de défaut. F2 est un fusible réarmable, qui ne met la carte hors fonction qu'au moment où le défaut est présent. La maintenance des cartes avec le fusible F2 sera donc moins importante.

L'alimentation de la carte peut être réalisée par une alimentation secteur murale d'une puissance au moins égale à 6 VA, non régulé, et fournissant 12 V sous 500 mA. De préférence, la tension de 12V en charge doit être garantie pour conserver une vitesse correcte du moteur 159 de fermeture et d'ouverture de la vanne ou boisseau. Si l'alimentation est trop faible, la vanne ne s'ouvrira pas complètement.

Une Led verte permet de visualiser la présence d'alimentation sur l'électronique.

Un connecteur miniKK huit broches permet de raccorder le moteur, le ventilateur, le bouton poussoir et le jack d'alimentation.

Dans une application multimédia, le diffuseur selon l'invention pourra être commandé par le port UBS d'un ordinateur.

Les fonctions de détrompage pourront être assurées alternativement par les reliefs d'accouplement des arbres d'entraînement 151 et 163 en vue de ne pouvoir associer que certains diffuseurs 102 avec certains supports 140.

L'une des grilles 134 pourra être venue de moulage avec le boisseau 120.

Dans le diffuseur, la puce pourra servir au stockage de diverses données telles que date de fabrication, code du produit, etc.

On pourra prévoir en sortie du support une buse amovible réglable en orientation de façon à obtenir la meilleure perception olfactive en fonction du positionnement de l'utilisateur.

Lorsque le diffuseur 2, 102 comporte une unique face sphérique, celle-ci pourra être prévue sur le corps 4, 104, le boisseau 20, 120 ayant une face externe cylindrique.

La ou les faces sphériques pourra être remplacée par une face à profil courbe dans un plan radial à l'axe de rotation et dont la courbure est toujours dans le même sens, c'est-à-dire sans inflexion.

Par ailleurs, dans un autre mode de réalisation, le diffuseur 2 pourra être utilisé directement, sans support 40. La diffusion du parfum se fera alors sans circulation d'air forcé. Le diffuseur pourra par exemple être porté autour du cou de l'utilisateur. L'étanchéité du diffuseur autorise même l'utilisateur à se baigner avec.

Les substances à diffuser peuvent être des parfums, par exemple pour l'animation olfactive d'un lieu, ou pour présenter des parfums dans un magasin. Il peut s'agir de phéromones permettant de repousser ou d'attirer des insectes ou des animaux. Le port du diffuseur sur l'utilisateur offre alors une bonne protection contre les insectes dangereux. Il peut s'agir d'une substance désodorisante, assainissante ou bactéricide.

Les orifices 36 pourront avoir une forme non circulaire, par exemple elliptique ou polygonale, les parois 34, 134 étant planes.

On pourra mettre en oeuvre les caractéristiques relatives aux orifices de la paroi retenant les granulés, indépendamment de la forme des faces du diffuseur.

En remplacement du ventilateur 44, 144, le dispositif 40, 140 pourra comprendre une source de gaz inerte comprimé.

Le dispositif de diffusion 40, 140 pourra comporter plusieurs modules comprenant chacun plusieurs diffuseurs 2, 102 définissant ainsi une matrice rectangulaire de diffuseurs 2, 102.

Le diffuseur pourra avoir des configurations variées très différentes de celles qui viennent d'être décrites.

Le diffuseur et le dispositif de diffusion selon l'invention permettent de diffuser des odeurs ou des parfums sans utiliser de solvant, notamment d'alcool, interdit par certaines législations nationales pour la diffusion d'odeur dans certaines circonstances, notamment en public.

## Revendications

1. Diffuseur de substance volatile (2 ; 102) comportant un corps (4 ; 104) présentant au moins une ouverture (11 ; 111), et un réservoir (20 ; 120) d'un produit (35) comprenant une substance volatile, le réservoir (20 ; 120) étant monté mobile dans le corps (4 ; 104) entre une position de diffusion permettant qu'un gaz circule à travers le produit, puis dans l'ouverture (11 ; 111) et une position fermée dans laquelle le produit (35) est isolé de l'ouverture (11 ; 111), le réservoir et le corps présentant des faces respectives (22, 16 ; 122, 116) s'étendant en regard l'une de l'autre, **caractérisé en ce que** l'une des faces (22, 16 ; 122) est sphérique et **en ce qu'**il comporte au moins un élément (19 ; 119) assurant une étanchéité entre le corps (4 ; 104) et le réservoir (20 ; 120) dans la position fermée.

2. Diffuseur selon la revendication 1, **caractérisé en ce que** la face sphérique est celle du réservoir (20 ; 120).

3. Diffuseur selon la revendication 1 ou 2, **caractérisé en ce que** le réservoir (20 ; 120) est mobile à rotation par rapport au corps (4 ; 104).

4. Diffuseur selon la revendication 3, **caractérisé en ce que** les faces (16, 22 ; 116, 122) présentent chacune une symétrie de révolution autour de l'axe de rotation (14 ; 114).

5. Diffuseur selon l'une quelconque des revendications 1 à 4, **caractérisé en ce que** les faces (16, 22 ; 116, 122) sont sphériques.

6. Diffuseur selon l'une quelconque des revendications 1 à 5, **caractérisé en ce que** le réservoir (122) présente une face interne sphérique (124).

7. Diffuseur selon l'une quelconque des revendications 1 à 6, **caractérisé en ce que** l'ouverture (11 ; 111) étant une première ouverture, le corps (4 ; 104) présente une deuxième ouverture (11 ; 111) adaptée pour qu'en position de diffusion, le gaz circule dans la deuxième ouverture (11 ;111), puis à travers le produit (35), puis dans la première ouverture (11 ; 111).

8. Diffuseur selon l'une quelconque des revendications 1 à 7, **caractérisé en ce que** le réservoir (20) comporte une cartouche amovible (32) comprenant le produit (35).

9. Diffuseur selon l'une quelconque des revendications 1 à 8, **caractérisé en ce que** le produit (35) comporte des granulés comprenant la substance volatile à l'état adsorbé.

10. Diffuseur selon l'une quelconque des revendications 1 à 9, **caractérisé en ce que** le réservoir (20 ; 120)comporte une paroi (34 ; 134) présentant des orifices (36) ayant chacun un contour conformé de sorte qu'il ménage un passage de gaz lorsqu'un granulé sphérique est en appui sur l'orifice.

11. Diffuseur selon la revendication 10, **caractérisé en ce que** les orifices (36) ont un contour circulaire gauche.

12. Diffuseur selon la revendication 10 ou 11, **caractérisé en ce que** la paroi (34 ; 134) est ondulée.

13. Dispositif de diffusion de substance volatile, **caractérisé en ce qu'**il comprend au moins un diffuseur (2 ; 102) selon l'une quelconque des revendications 1 à 12, et un support (40 ; 140) adapté à recevoir le diffuseur ou les diffuseurs (2 ; 102) simultanément.

14. Dispositif selon la revendication 13, **caractérisé en ce que** le support (40) comporte des moyens (159) pour commander la position du réservoir (20 ; 120) du ou de chaque diffuseur (2 ; 102).

15. Dispositif de diffusion selon l'une quelconque des revendications 13 à 14, **caractérisé en ce que** le support (40 ; 140) comporte des moyens (44 ; 144) pour mettre un gaz en circulation à travers le ou chaque diffuseur (2 ; 102) en position de diffusion.

16. Dispositif de diffusion selon la revendication 15, tel qu'un orgue, **caractérisé en ce que** le support (40) comprend au moins deux diffuseurs (2), les moyens de circulation de gaz (44) étant communs aux diffuseurs.

17. Dispositif de diffusion selon l'une quelconque des revendications 13 à 16, **caractérisé en ce qu'**il comporte des moyens de détrompage (145, 147) pour la réception du diffuseur (2 ; 102) dans au moins une position prédéterminée dans le support (40 ; 140).

18. Dispositif de diffusion selon l'une quelconque des revendications 13 à 17, **caractérisé en ce qu'**il comporte des moyens de détrompage (143, 149) pour la réception dans le support (140) d'au moins un diffuseur prédéterminé (102).

## Claims

1. Diffuser (2; 102) of a volatile substance comprising a body (4; 104) which has at least one opening (11; 111) and a reservoir (20; 120) for a product (35) containing a volatile substance, the reservoir (20; 120) being mounted so that it can move in the body (4; 104) between a diffusing position which allows a gas to flow through the product, then into the opening (11; 111), and a closed position in which the product (35) is isolated from the opening (11; 111), the reservoir and the body having respective faces (22, 16; 122, 116) extending facing each other, **characterized in that** one of the faces (22, 16; 122) is spherical and **in that** it comprises at least one element (19; 119) which makes a seal between the body (4; 104) and the reservoir (20; 120) in the closed position.

2. Diffuser according to claim 1, **characterized in that** the spherical face is that of the reservoir (20; 120).

3. Diffuser according to claim 1 or 2, **characterized in that** the reservoir (20; 120) can move in terms of rotation with respect to the body (4; 104).

4. Diffuser according to claim 3, **characterized in that** the faces (16, 22; 116, 122) each have symmetry of revolution about the axis of rotation (14; 114).

5. Diffuser according to any one of claims 1 to 4, **characterized in that** the faces (16, 22; 116, 122) are spherical.

6. Diffuser according to any one of claims 1 to 5, **characterized in that** the reservoir (122) has a spherical interior face (124).

7. Diffuser according to any one of claims 1 to 6, **characterized in that** with the opening (11; 111) being a first opening, the body (4; 104) has a second opening (11; 111) designed so that, in the diffusing position, the gas flows into the second opening (11; 111), then through the product (35), then into the first opening (11; 111).

8. Diffuser according to any one of claims 1 to 7, **characterized in that** the reservoir (20) comprises a removable cartridge (32) containing the product (35).

9. Diffuser according to any one of claims 1 to 8, **characterized in that** the product (35) comprises granules containing the volatile substance in the adsorbed state.

10. Diffuser according to any one of claims 1 to 9, **characterized in that** the reservoir (20; 120) has a wall (34; 134) exhibiting orifices (36), each having a contour shaped such that it forms a passage for gas when a spherical granule is resting against the orifice.

11. Diffuser according to claim 10, **characterized in that** the orifices (36) have a skewed circular contour.

12. Diffuser according to claim 10 or 11, **characterized in that** the wall (34; 134) is undulating.

13. Device for diffusing a volatile substance, **characterized in that** it comprises at least one diffuser (2; 102) according to any one of claims 1 to 12, and a support (40; 140) designed to accept the diffuser or diffusers (2; 102) simultaneously.

14. Device according to claim 13, **characterized in that** the support (40) comprises means (159) for controlling the position of the reservoir (20; 120) of the or each diffuser (2; 102).

15. Diffusion device according to either one of claims 13 and 14, **characterized in that** the support (40; 140) comprises means (44; 144) for circulating a gas through the or each diffuser (2; 102) in the diffusing position.

16. Diffusion device according to claim 15, such as an organ pipe, **characterized in that** the support (40) comprises at least two diffusers (2), the gas-circulating means (44) being common to the diffusers.

17. Diffusion device according to any one of claims 13 to 16, **characterized in that** it comprises polarizing means (145, 147) for accepting the diffuser (2; 102) in the support (40; 140) in at least one predetermined position.

18. Diffusion device according to any one of claims 13 to 17, **characterized in that** it comprises polarizing means (143, 149) for accepting at least one predetermined diffuser (102) in the support (140).

## Patentansprüche

1. Diffusor flüchtiger Substanzen (2; 102), der einen Körper (4; 104) mit wenigstens einer Öffnung (11; 111) und einen Behälter (20; 120) eines Produktes (35) umfaßt, das eine flüchtige Substanz umfaßt, wobei der Behälter (20; 120) beweglich im Körper (4; 104) zwischen einer Diffusionsposition, die einem Gas die Zirkulation über das Produkt, dann in die Öffnung (11; 111) ermöglicht, und einer geschlossenen Position angebracht ist, in der das Produkt (35) von der Öffnung (11; 111) isoliert ist, wobei der Behälter und der Körper jeweilige Flächen (22, 16; 122, 116) aufweisen, die einander gegenüberliegen, **dadurch gekennzeichnet, daß** eine der Flächen (22, 16; 122) kugelförmig ist und daß sie wenigstens ein Element (19; 119) aufweist, das eine Dichtigkeit zwischen dem Körper (4; 104) und dem Behälter (20; 120) in der geschlossenen Position sicherstellt.

2. Diffusor nach Anspruch 1, **dadurch gekennzeichnet, daß** die kugelförmige Fläche diejenige des Behälters (20; 120) ist.

3. Diffusor nach Anspruch 1 oder 2, **dadurch gekennzeichnet, daß** der Behälter (20; 120) drehbeweglich bezüglich des Körpers (4; 104) ist.

4. Diffusor nach Anspruch 3, **dadurch gekennzeichnet, daß** die Flächen (16, 22; 116, 122) jeweils eine Rotationssymmetrie um die Rotationsachse (14; 114) aufweisen.

5. Diffusor nach einem der Ansprüche 1 bis 4, **dadurch gekennzeichnet, daß** die Flächen (16, 22; 116, 122) kugelförmig sind.

6. Diffusor nach einem der Ansprüche 1 bis 5, **dadurch gekennzeichnet, daß** der Behälter (122) eine kugelförmige Innenfläche (124) aufweist.

7. Diffusor nach einem der Ansprüche 1 bis 6, **dadurch gekennzeichnet, daß** die Öffnung (11; 111) eine erste Öffnung ist, der Körper (4; 104) eine zweite Öffnung (11; 111) aufweist, die derart ausgestaltet ist, daß in der Diffusionsposition das Gas in die zweite Öffnung (11; 111), dann über das Produkt (35), dann in die erste Öffnung (11; 111) zirkuliert.

8. Diffusor nach einem der Ansprüche 1 bis 7, **dadurch gekennzeichnet, daß** der Behälter (20) eine austauschbare Kartusche (32) aufweist, die das Produkt (35) enthält.

9. Diffusor nach einem der Ansprüche 1 bis 8, **dadurch gekennzeichnet, daß** das Produkt (35) Körner aufweist, welche die flüchtige Substanz im adsorbierten Zustand aufnehmen.

10. Diffusor nach einem der Ansprüche 1 bis 9, **dadurch gekennzeichnet, daß** der Behälter (20; 120) eine Wandung (34; 134) aufweist, die Öffnungen (36) mit jeweils einer Kontur aufweisen, die derart angepaßt ist, daß sie einen Gasdurchlaß freilegt, wenn ein kugelförmiges Körnchen an der Öffnung anliegt.

11. Diffusor nach Anspruch 10, **dadurch gekennzeichnet, daß** die Öffnungen (36) eine linkszirkulare Kontur aufweisen.

12. Diffusor nach Anspruch 10 oder 11, **dadurch gekennzeichnet, daß** die Wandung (34; 134) gewellt ist.

13. Vorrichtung zur Diffusion flüchtiger Substanzen, **dadurch gekennzeichnet, daß** sie wenigstens einen Diffusor (2; 102) nach einem der Ansprüche 1 bis 12 aufweist, und einen Träger (40; 140), der ausgestaltet ist, den Diffusor oder die Diffusoren (2; 102) gleichzeitig aufzunehmen.

14. Vorrichtung nach Anspruch 13, **dadurch gekennzeichnet, daß** der Träger (40) Mittel (159) zum Steuern der Position des Behälters (20; 120) des oder jedes Diffusors (2; 102) aufweist.

15. Diffusionsvorrichtung nach einem der Ansprüche 13 bis 14, **dadurch gekennzeichnet, daß** der Träger (40; 140) Mittel (44; 144) aufweist, um ein Gas in Zirkulation über den oder jeden Diffusor (2; 102) in Diffusionsposition zu bringen.

16. Diffusionsvorrichtung nach Anspruch 15, wie eine Orgel, **dadurch gekennzeichnet, daß** der Träger (40) wenigstens zwei Diffusoren (2) umfaßt, wobei die Gaszirkulationsmittel (44) für die Diffusoren gemein sind.

17. Diffusionsvorrichtung nach einem der Ansprüche 13 bis 16, **dadurch gekennzeichnet, daß** sie Unverwechselbarkeitsmittel (145, 147) für die Aufnahme des Diffusors (2; 102) in wenigstens einer vorbestimmten Position im Träger (40; 140) umfaßt.

18. Diffusionsvorrichtung nach einem der Ansprüche 13 bis 17, **dadurch gekennzeichnet, daß** sie Unverwechselbarkeitsmittel (143, 149) für die Aufnahme wenigstens eines vorbestimmten Diffusors (102) im Träger (140) umfaßt.
